Europäisches Patentamt

European Patent Office

Office européen des brevets

(19)

(11) Veröffentlichungsnummer: **0 249 594**
**B1**

(12) **EUROPÄISCHE PATENTSCHRIFT**

(45) Veröffentlichungstag der Patentschrift: 04.04.90

(51) Int. Cl.⁵: **A 61 F 2/30,** A 61 F 2/32,
A 61 F 2/34, A 61 B 17/56

(21) Anmeldenummer: 85905529.5

(22) Anmeldetag: 05.11.85

(88) Internationale Anmeldenummer:
PCT/HU85/00062

(87) Internationale Veröffentlichungsnummer:
WO 86/05679 09.10.86 Gazette 86/22

(54) **KLEBSTOFFLOSE GELENKPFANNENKONSTRUKTION FÜR PROTHESEN, SOWIE VORRICHTUNG ZUR IMPLANTATION.**

(30) Priorität: 29.03.85 HU 118685

(43) Veröffentlichungstag der Anmeldung:
23.12.87 Patentblatt 87/52

(45) Bekanntmachung des Hinweises auf die
Patenterteilung:
04.04.90 Patentblatt 90/14

(84) Benannte Vertragsstaaten:
AT CH DE FR GB LI SE

(56) Entgegenhaltungen:
CH-A- 453 570
DE-A-2 530 254
DE-A-2 544 918
DE-A-3 310 944
DE-B-2 260 839
DE-B-2 365 022

(73) Patentinhaber: INNOMARK MARKETING-
TANACSADO FEJLESZTÖ VALLALAT
Parisi u. 3
H-1052 Budapest V. (HU)

(72) Erfinder: BADO, Zoltán
Ifjuság sétány 12
H-6600 Szentes (HU)
Erfinder: TARI, Gábor
Hársfa u. 16
H-6640 Csongrád (HU)
Erfinder: JUHASZ, Imre
Felszabadulás u. 61/a
H-6800 Hódmezovásárhely (HU)

(74) Vertreter: Patentanwälte Viering & Jentschura
Steinsdorfstrasse 6
D-8000 München 22 (DE)

**Beschreibung**

Gegenstand der Erfindung sind eine Gelenkpfannenkonstruktion für Prothesen, insbesondere Hüftgelenksprothesen, die mit einem aus gewebefreundlicher Metallegierung gefertigten kegeligen Pfannenkorb versehen ist und ohne den Einsatz von Klebstoff mechanisch in der Gelenkgrube befestigt werden kann, sowie eine Vorrichtung zur Implantation.

Auf der ganzen Welt werden täglich etwa 4000 künstliche Gelenke, in erster Linie Hüftgelenke implantiert. Diese Zahl nimmt laufend zu, da proportional zur rapiden Erhöhung des menschlichen Lebensalters auch die abnutzungsbedingten Erkrankungen der Gelenke häufiger werden. Die dynamische Entwicklung der Chirurgie und ihrer Randgebiete erhöht die Zahl der Eingriffe weiter. Gleichzeitig zeichnen sich auch die zahlreichen Schwachstellen der nunmehr zu einem Routineverfahren entwickelten Operationen immer markanter ab.

Die Gelenke bestehen aus zwei Komponenten: dem kugelförmigen Gelenkkopf des Halsteiles des in Betracht kommenden Knochens, der sich in das hohl ausgebildete "Gegenstück" des Knochens, in die Gelenkgrube/Gelenkpfanne/einfügt. Beide Gelenkflächen der durch verschiedene Krankheitsursachen destruierten Gelenke müssen ersetzt werden. Im Falle des Hüftgelenkes legt z.B. der Chirurg die Körpergegend frei und entfernt zuerst den Kopf- und Halsteil des Schenkelknochens bis zur Intertrochanterebene; dann wird in der so erschlossenen Markhöhle des Schenkelbeines das entfernte Knochenteil durch eine biomechanisch zweckdienlich ausgebildete, aus gewebefreundlichem Metall gefertigte Prothese ersetzt, die aus unterschiedliche Abmessungen und Formen aufweisenden Varianten optimal ausgewählt werden kann. Die Prothese wird mittels Knochenzements bzw. mechanisch fixiert und hiernach wird in der ausgehöhlten Grube des Hüftbeines das aus gewebefreundlichem Kunststoff gefertigte Gegenstück, die künstliche Gelenkpfanne befestigt. Durch Zusammenfügen dieser beiden kommt das "Kugelgelenk" d.h. die Hüftgelenkprothese zustande.

Unter den mit der vorstehend vereinfacht beschriebenen Operation verbundenen zahlreichen recht komplizierten Teilproblemen ist als eine der am meisten gefürchteten aber häufigen Komplikationen die Lockerung der Hüftgelenkspfanne und hiernach das Herausreissen des ganzen künstliches Gelenkes aus ihrer Einsatzstelle zu erwähnen. Bei biomechanischer Betrachtung der Erscheinung ist eine Aufgliederung des Wortes selbst richtungsweisend, im weit verzweigten Labyrinth der Probleme: die ungenügende Fixierung und demzufolge Lockerung der Prothesenpfanne kann annehmbarerweise auf zwei Hauptursachen zurückgeführt werden: auf eine biologische bzw. mechanische. Die biologische Ursache ist in erster Linie in den Eigenschaften des Klebstoffes zu suchen. Zu diesem Zweck wird Methylmetakrilat verwendet, das in seiner mit einem Katalysator bei Raumtemperatur polymerisierenden Form zum Teil nicht vollkommen polymer ist, dessen freie Monomere gewebezerstörend sind und das nicht einmal bei "Raumtemperatur" reagiert, da die exotherme Wärmebildung auch 75°C erreichen kann und so die lebenden Proteine denaturiert. Auch eine kalte Flüssigkeitsspülung während der Operation bietet hier nicht viel Abhilfe, da sowohl die Kunststoffpfanne als auch das Akrylat selbst über gute Wärmedämmung verfügen. Das Ergebnis ist die Zerstörung der Knochensubstanz und demzufolge die Lockerung der Prothese.

Die Erklärung der mechanischen Ursache liegt in den anatomischen Verhältnissen: während das trichterartig sich erweiternde Ende des Schenkelröhrenknochens die Möglichkeit einer verhältnismässig guten und stabilen Einfügung für die Prothese bietet, ist die Hüftgrube flach konkav, sodass sich darin die besser oder schlechter einklebbare und obendrein halbrunde d.h. nicht formschlüssige Prothese unter Einwirkung der übrigens nicht in ihrer Achslinie wirkenden und sich unzählige Male wiederholenden, durch das Körpergewicht bedingten Scher- und Druckkräfte leicht lockert.

Der gleiche Sachverhalt liegt im wesentlichen auch bei den sonstigen Gelenkpfannenprothesen vor. Weltweit wird in zunehmender Masse versucht, die biologischen Mängel einfach durch Weglassen des Knochenzements zu beseitigen und die Prothesen, wo dies nur möglich ist — mechanisch zu fixieren. Dies erscheint wie bereits ausgeführt, bei den Gelenkköpfen als lösbar, wogegen bei den Gelenkgruben schwerwiegende Schwierigkeiten verursacht werden.

Aus diesem Grunde wird mit neuen Entwicklungslösungen experimentiert, die auf eine Beseitigung der vorgenannten Schwierigkeiten abzielen. Die eine derartige Lösung ist, die Aussenfläche des die Gelekpfanne aufnehemenden Metallpfannenkorbes mit biologisch gewebefreundlicheren, sogenannten bioaktiven Stoffen zu überziehen, die mit der Knochensubstanz sozusagen verwachsen und dadurch die mechanische Befestigung sichern.

Nach einer anderen Ausführungslösung wird der Pfannenkorb mit einer unregelmässigen, porösen "bioaktiven" Oberfläche versehen, die mit der Zeit ebenfalls in die zur Einbettung dienende Knochenoberfläche hineinwächst.

Obwohl diese Lösungen tatsächlich eine sichere Verbindung als die früheren Ausführungen herbeiführen, besteht ihr grundlegender Nachteil darin, dass der operierte Patient den langen Vorgang des Hineinwachsens, der auch mehrere Wochen andauern kann, im Bett liegend erwarten muss.

Mit Rücksicht darauf, dass die Implantation der Prothese in der Mehrzahl in einem fortgeschrittenen Alter erfolgt, erhöht die Bettgebundenheit der Patienten nach der Operation das Risiko derselben in grossem Masse/Lungenentzündung, Thrombose, Wundliegen usw./.

Es werden auch Praktiken angewandt, bei denen auf der Aussenfläche des Pfannenkorbes Schraubengewinde bzw. Gewindesegmente ausgebildet und diese in das vorbereitete Pfannenbett eingeschraubt werden.

Die mittels Schraubengewinde erfolgende Fixierung kann auf die Weise vorgenommen werden, dass in die Pfannengrube vorhergehend ein Gewinde geschnitten wird, bzw. eine das Gewinde selbsttätig schneidende Ausführung zur Anwendung gelangt.

Ein derartiger mit Gewinde versehender Pfannenkorb ist in der Mehrzahl kegelförmig ausgebildet und kann so nicht in die Beckenhöhle eindringen. Der Kegelwinkel gewährleistet gleichzeitig auch die Verkeilung, weshalb diese Ausführung auch einem Herauskippen besser als die früher angewandten Lösungen widersteht. Unverändert ist jedoch der Nachteil, dass sie aus der übrigens bereits ledierten spongiösen Knochensubstanz sehr leicht herausreisst.

Zweck vorliegender Erfindung ist deshalb eine Konstruktion zu schaffen, die eine ohne Klebemittel erfolgende mechanische Befestigung des Gelenkpfannenteiles der verschiedenen Prothesen, insbesondere der Hüftprothesen in der Gelenkgrube auf die Weise ermöglicht, dass dabei das Einsetzen ausserordentlich einfach vorzunehmen, die Verbindung vollkommen zuverlässig und nötigenfalls einfach lösbar ist.

Die gestellte Aufgabe wurde erfindungsgemäss dadurch gelöst, dass der die Gelenkpfanne tragende Pfannenkorb mit Aussparungen in der Mantellinienrichtung versehen ist und in den Aussparungen L-förmige Anker, um die zur Tangente des Pfannenkorbmantels parallelen geometrischen Achse schwenkbar so eingefügt sind, dass der kürzere Teil des Ankers bei dem Pfannenkorbende mit dem kleineren Durchmesser angeordnet und am äusseren Ende als eine Spitze bzw. scharfe Kralle ausgebildet ist, wobei die Stärke des längeren Ankerteiles gleich der Wandstärke des Pfannenkorbes und seine Länge gleich der Länge der Aussparung ist.

Die Seitenwände der Aussparungen können parallel zum Radius verlaufen, ihre zum Boden des Pfannenkorbes zu liegende Fläche ist bogenförmig, wobei auf ihrer zur Öffnung des Pfannenkorbes zu liegenden Flächen eine Bettungsrinne oder Rippe ausgebildet ist. An diese fügt sich zur Sicherung der Schwenkbarkeit der Anker die an dem zur Kralle entgegengesetzten Ende des längeren Teiles der Anker ausgebildete Bettungsrippe bzw. Bettungrinne.

Bei einer vorteilhaften Ausführungsform der erfindungsgemässen Konstruktion ist auf der zur Öffnung des Pfannenkorbes liegenden Fläche der Aussparungen ein konvexes Zylindermantelsegment mit einer zur Richtung der Tangente der äusseren Mantelfläche parallelen geometrischen Achslinie und in der unteren Fläche des längeren Teiles der Anker ist zugleich ein konkaves Zylindermantelsegment mit einer zur Längsachse der Kralle senkrechten geometrischen Achslinie ausgebildet.

Der Pfannenkorb selbst ist auch innen kegelförmig ausgebildet und in seinem Boden ist eine zentrale Gewindebohrung vorgesehen. Die Gelenkpfanne ist mit einem sich in die Gewindebohrung hineinfügenden zentralen Gewindezapfen versehen.

Die an den Ankern befindliche/n/Kralle/n/ können mehrkantig und/oder mit mehreren spitzen bzw. mit Rippen und/oder Nuten versehen sein. Ihre untere Fläche kann unter anderem bogenförmig oder spiralförmig ausgebildet sein.

Die zur Befestigung der erfindungsgemässen Konstruktion dienende Vorrichtung besteht aus einer Welle, die an ihrem einen Ende mit einem sich in das im Boden des Pfannenkorbes befindliche Gewinde einfügendem Gewinde, an ihrem anderen Ende mit einem Griff versehen ist, auf diese Welle ist eine sich mit einem Schraubgewinde einfügende Rohrwelle aufgezogen, wobei an dem griffseitigen Ende der Rohrwelle ein Antriebsrad, an dem sich an den Pfannenkorb fügenden Ende hingegen ein bogenförmiger bzw. eine Kugelfläche aufweisender Spanneinsatz angeordnet sind.

Die erfindungsgemässe Ausführung ermöglicht eine mehr Sicherheit bietende Befestigung des Pfannenkorbes als alle bisherigen Ausführungen. Die sich in die Knochensubstanz vertiefenden Krallen ziehen im Verlaufe ihres Eindringens den Pfannenkorb sozusagen in die Knochengrube und die in den Korb eingesetzte Gelenkpfanne verhindert ein Herauslösen der Krallen. Der Pfannenkorb ist auf diese Weise im Verlaufe seines Einsatzes vollkommen festgebettet, eine Lockerung und ein Herausfallen desselben ist vollkommen ausgeschlossen.

Gleichzeitig kann nötigenfalls der Pfannenkorb ganz einfach aus seinem Platz herausgehoben werden, wenn nach Entfernen der Gelenkpfanne die Anker zurückgezogen werden. Für diesen Zweck können die Anker gegebenenfalls Bohrungen oder sonstige Mittel enthalten.

Die Einzelheiten der Erfindung werden anhand Ausführungsbeispiele mit Hilfe einer Zeichnung beschrieben. Auf der Zeichnung zeigen

Fig. 1 die Anordnung der Bauelemente der Hüftgelenksprothese in dem Schenkelknochen bzw. Hüftbein;

Fig. 2 den Schnitt einer zweckdienlichen Ausführungsform des erfindungsgemässen Pfannenkorbes;

Fig. 3 die Ansicht des in Fig. 2 dargestellten Pfannenkorbes;

Fig. 4 die Ansicht einer Gelenkpfanne;

Fig. 5 den Schnitt der zusammengebauten Gelenkpfannen-Pfannenkorbkonstruktion;

Fig. 6 die Ansicht der in Fig. 5 dargestellten Konstruktion;

Fig. 7 eine Ausführungsform des in der erfindungsgemässen Ausführung verwendeten Ankers;

Fig. 8 eine andere Ausführungsform des Ankers;

Fig. 9 eine weitere Ausführungsform des Ankers;

Fig. 10 die Ansicht einer Variante der erfindungsgemässen Konstruktion mit den in Fig. 7 dargestellten Ankern;

Fig. 11 das Ende der zum Ein- und Ausschrauben der Gelenkpfanne dienenden Vorrichtung;

Fig. 12 eine zweckdienliche Ausführungsform der zum Einsetzen des Pfannenkorbes dienenden Vorrichtung zum Teil im Schnitt;

Fig. 13 die Schnittzeichnung des Kopfteiles der Vorrichtung und des Pfannenkorbes und

Fig. 14 die Ansicht der in Fig. 12 dargestellten Vorrichtung.

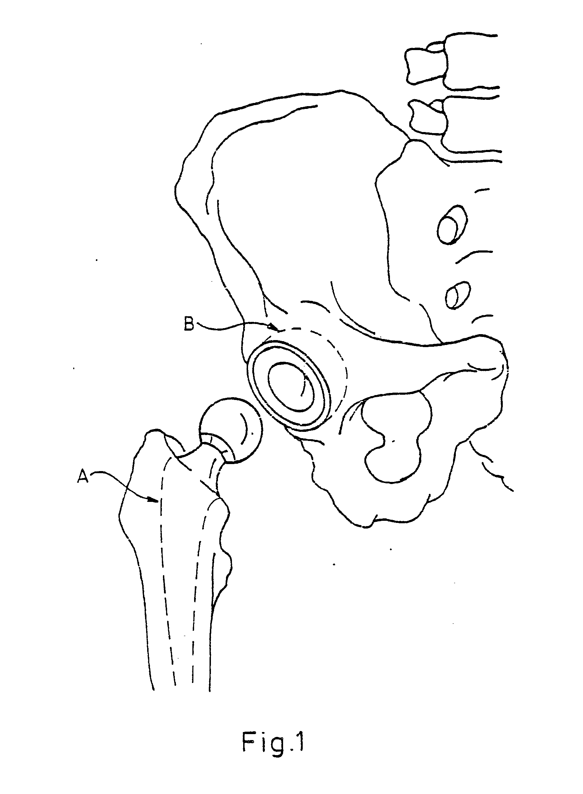

In Fig. 1 ist zu sehen, dass die Hüftgelenkprothesen aus zwei Teilen bestehen: aus dem in das Schenkelbein befestigten Femurteil A und der in das Hüftbein eingesetzten Gelenkpfannenkonstruktion B. Vorliegende Erfindung bezieht sich auf die Gelenkpfannenkonstruktion, bestehend aus dem metallenen Pfannenkorb und der darin befestigten Gelenkpfanne.

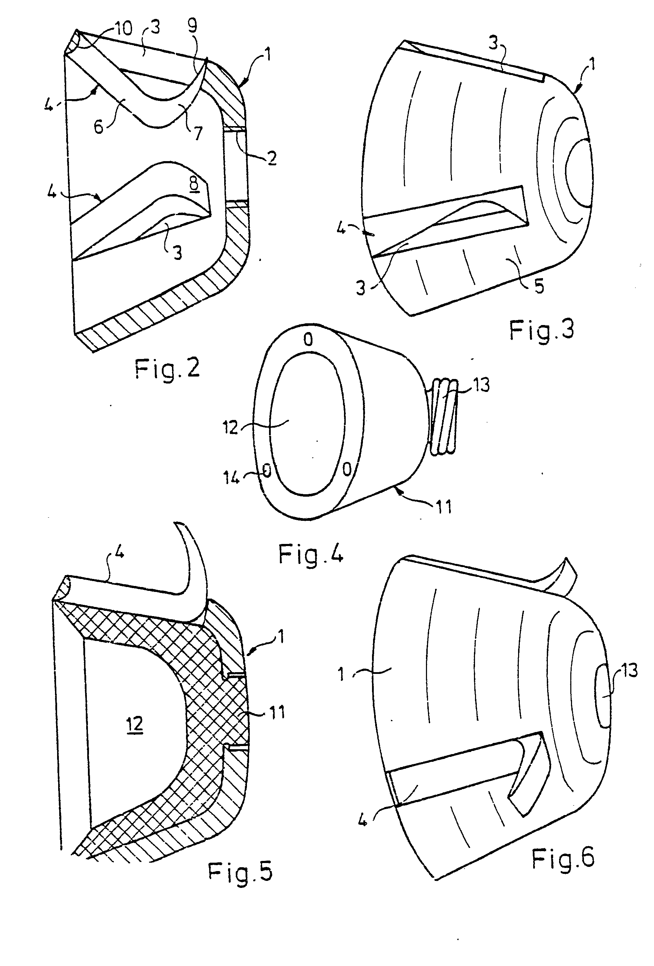

In Fig. 2 und 3 ist eine vorteilhafte Ausführung des erfindungsgemässen Pfannenkorbes zu sehen. Der Pfannenkorb 1 ist im wesentlichen ein schalenförmiger Metallkörper, der aus einer gewebefreundlichen Legierung hergestellt wird. Aussen und innen kegelförmig ausgebildet ist der Korb in seinem Bodenteil mit einer zentralen Gewindebohrung 2 ausgestaltet. Seitlich sind entlang der Mantellinie verlaufende Aussparungen 3 vorgesehen und in diesen die Anker 4 angeordnet. Die äussere Mantelfläche ist im Interesse der sicheren Befestigung mit den Rippen 5 versehen.

Die Anker 4 sind L-förmig ausgebildet und bestehen aus dem Schaft sowie den Krallen 7. Die obere Fläche 8 der Kralle 7 hat eine bogenförmige, die untere Fläche 9 eine spiralförmige Ausbildung.

Die Seitenwände der Aussparung 3 verlaufen parallel zur radialen Richtung des Kegelstumpfes, ihre obere Begrenzungsfläche ist derart bogenförmig ausgebildet, dass sie sich an die obere Fläche 8 der Krallen 7 der Anker 4 anfügt. Demzufolge ist der Bogen der oberen Fläche der Krallen 7 und der Bogen der oberen Fläche der Aussparungen 3 mit dem gleichen Radius ausgebildet.

Am unteren Teil der Aussparungen 3 sind die Bettungsrippen 10 ausgestaltet. Auf diesen liegen die Schäfte der Anker 4 auf, da an ihrem unteren Teil eine bogenförmige Hohlkehle ausgebildet ist.

In Fig. 2 und 3 befinden sich die Anker 4 in der Grundstellung, d.h. die Krallen 7 reichen nicht über die äussere Mantellinie des Pfannenkorbes 1 hinaus. Auf diese Weise kann der Pfannenkorb 1 leicht in die Gelenkgrube eingesetzt werden.

In Fig. 4 ist das andere Bauelement der Konstruktion die Gelenkpfanne 11 zu sehen, die aus gewebefreundlichem form-beständigem und verschliessfestem Kunststoff, z.B. aus kohlenfadenverstärktem Polyäthylen RCH1000 hergestellt werden. Ihr Aussenmantel liegt auf der Innenfläche des Pfannenkorbes 1 auf, ihr Hohlraum hat eine Kugeloberfläche und dient zur Aufnahme des anderen Bauelementes der Prothese bzw. zur Sicherung der räumlichen Gelenkfunktion.

Die Gelenkpfanne 11 ist mit einem Gewindezapfen 13 versehen, der sich in die Gewindebohrung 2 des Pfannenkorbes hineinfügt. Das Ein- und Ausschrauben kann mit Hilfe der am Rand des Hohlraumes 12 vorgesehenen Bohrungen 14 vorgenommen werden.

In den Fig. 5 und 6 ist der in den Fig. 2 und 3 dargestellte Pfannenkorb 1 in dem Zustand zu sehen, in dem er in der Gelenkgrube fixiert angeordnet ist. Ersichtlich ist, dass die Schäfte 6 der Anker 4 in dieser Stellung mit der Wand des Pfannenkorbes 1 zusammenfallen und die Krallen 7 in ihrer ganzen Länge aus der Mantelfläche hervorstehen und auf diese Weise die Pfannenkonstruktion festhalten. Ein Zurückschwenken oder eine Lockerung der Anker 4 wird durch die in den Pfannenkorb 1 eingeschraubte Gelenkpfanne in vollem Ausmasse verhindert. In dieser Stellung ist zu ersehen, dass die Stärke der Schäfte 6 der Anker 4 die Wandstärke des Pfannenkorbes 1 auf keinen Fall überschreiten darf.

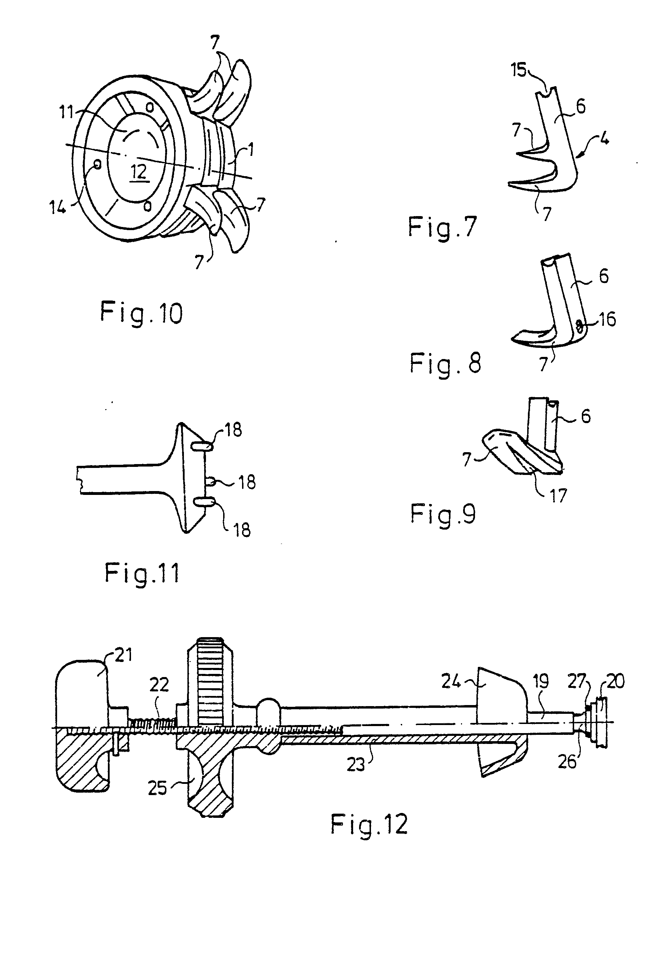

Mit Hilfe der in dem dargestellten Ausführungsbeispiel verwendeten, in ihrer Konstruktion ausserordentlich einfachen Anker kann die Befestigung des Pfannenkorbes mit voller Sicherheit gewährleistet werden. In gewisen Fällen kann jedoch auch der Einsatz von besonderen Ankern zweckdienlich werden. Derartige Spezialanker zeigen die Fig. 7, 8 und 9.

Der in Fig. 7 dargestellte Anker 4 ist zur Erhöhung der Sicherheit der Befestigung mit zwei Krallen 7 versehen.

Die Kralle 7 des in Fig. 8 dargestellten Ankers ist an ihrer unteren Fläche 9 mit einer Rippe versehen, an der Aussenseite des Schaftes 6 hingegen ist der Hohlraum 16 eingearbeitet, der das Zurückziehen der Anker 4 erleichtert, wenn der Pfannenkorb aus irgendeinem Grunde wieder aus der Gelenkgrube herausgenommen werden muss.

In Fig. 9 ist wieder eine weitere Ankerausführung zu sehen, die Kralle 7 ist in diesem Falle in einer verhältnismässig breiten Spitze ausgeführt und in ihrer oberen Fläche 8 ist die Nut 17 vorgesehen.

Fig. 10 zeigt einen Pfannenkorb 1, der mit den in Fig. 7 dargestellten doppelkralligen Ankern 4 ausgerüstet ist.

Zweckdienlich werden sowohl der Pfannenkorb als auch die Gelenkpfanne 11 sowie die Anker 4 ebenfalls in verschiedenen Grössen gefertigt, ähnlich den üblichen Prothesenbaureihen.

Die verschiedenen Typen der Anker 4 sind zweckdienlicherweise austauschbar und so kann stets die zum gegebenen Fall optimale Variante ausgewählt und nötigenfalls können sogar in einem einzigen Pfannenkorb mehrere unterschiedliche Krallen angeordnet werden.

In den Fig. 11 und 12 sind die zum Einsetzen bzw. zum Zusammenbau der erfindungsgemässen Gelenkpfannenkonstruktion verwendbaren Vorrichtungen/Werkzeuge/zu sehen. Mit Hilfe des in Fig. 1 dargestellten Werkzeuges kann die Gelenkpfanne 11 in den Pfannenkorb 1 mittels der in die Bohrungen 14 hineinpassenden Stifte 18 eingeschraubt werden.

Fig. 12 zeigt die zum Einsetzen und Fixieren des Pfannenkorbes in der Gelenkgrube dienende Vorrichtung, diese enthält eine Welle 19, an deren einem Ende ein mit Gewinde versehener Kopfteil ausgebildet ist. Der mit Gewinde versehene Kopfteil 20 fügt sich in die Gewindebohrung 2 des Pfannenkorbes 1 hinein.

Am anderen Ende der Welle 19 ist der Griff 21 angeordnet und unmittelbar davor ist das Schraubengewinde 22 ausgebildet.

Auf die Welle 19 ist die Rohrwelle 23 aufgezogen. Am einen Ende derselben ist der Druckeinsatz 24, am anderen Ende das Antriebsrad 25 angeordnet. Das Antriebsrad 25 kann im Vergleich zur rohrwelle 23 frei umlaufen und in seinem Inneren ist ein auf dem Schraubengewinde 22 der Welle 19 sitzendes Innengewinde ausgebildet. Auf diese Weise bewirkt ein Festhalten der Welle 19 und das Verdrehen des Antriebsrades 25 eine in axialer Richtung erfolgende Bewegung des Druckeinsatzes 24.

Die Welle 19 ist vor dem mit Gewinde versehenen Kopfteil 20 mit einem Halsteil 26 versehen und zwischen dem Halsteil 26 und dem mit Gewinde versehenen Kopfteil 20 ist der Gummiring 27 angeordnet.

Das Einsetzen der erfindungsgemässen Gelenkpfannenkonstruktion erfolgt in nachstehender Weise:

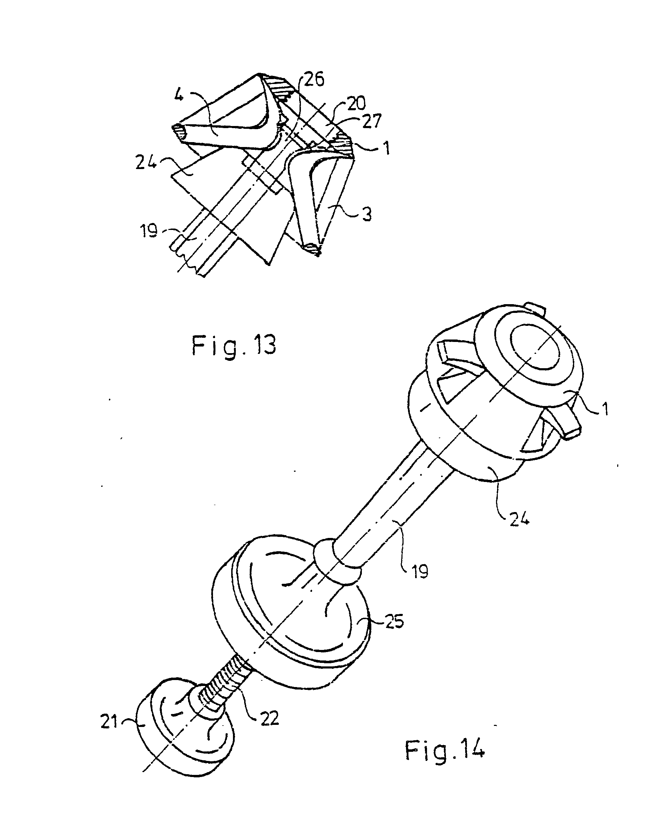

Der ausgewählte Pfannenkorb 1 wird auf den Gewindekopfteil 20 der Vorrichtung aufgeschraubt und dann werden in die Aussparungen 3 die dem Zweck entsprechend ausgewälten Anker auf die in Fig. 13 dargestellte Art und Weise eingesetzt. Das Einschwenken der Anker 4 in die Grundstellung ermöglicht der auf der Welle 19 vorgesehene Halsteil 26 und die Anker 4 werden in dieser Stellung durch den Gummiring 26 gegen Herausfallen gesichert.

Die so zusammengebaute Einheit wird in die mittels eines Sonderwerkzeuges auf Mass geformte leere Gelenkgrube des Hüftbeines eingepasst und bei Festhalten des Griffes 21 wird das Antriebsrad 25 zu drehen begonnen. Hierbei schwenkt der am Ende der Rohrwelle 23 befindliche Druckeinsatz 24 die Anker 4 allmählich auf die in Fig. 14 sichtbare Weise nach Aussen. Im Laufe dieser Bewegung werden die Krallen 7 in den Markbestand des Hüftbeines auf die Weise hineingepresst, dass dabei ihre spiralförmige untere Fläche 9 den Pfannenkorb 1 sozusagen in die Gelenkgrube hineinzieht.

Bei der vorgestellten zweckdienlichen Ausführungsform sind die Aussparungen 3 bzw. Anker 4 so auf dem Mantel des Pfannenkorbes 1 angeordnet, dass bei entsprechender Einstellung die Fixierung in anatomisch bestimmten Richtungen in Richtung des Hüftbeines, des Sitzbeines und des Schambeines, d.h. in Richtungen erfolgt, in denen der Knochenbestand verhältnismässig stark und dick ist.

Infolge der genannten Ausführung des Aussenmantels des Pfannenkorbes sowie der Anker verkeilt der Pfannenkorb auch allein in der Gelenkgrube. Nach dem die Verbindung zustandegekommen ist, wird der mit Gewinde versehene Kopfteil 20 der Welle 19 aus dem Pfannenkorb 1 herausgeschraubt und an seine Stelle die Gelenkpfanne 11 mit Hilfe der der in Fig. 11 dargestellten Vorrichtung/Werkzeug/eingeschraubt.

Aus vorstehender Beschreibung ist gut zu ersehen, was die Versuche auch eindeutig bewiesen haben: nicht nur das Einsetzen der erfindungsgemässen Konstruktion ist ausserordentlich einfach und schnell durchführbar, sondern sie gewährleitet auch eine wesentlich stärkere Verbindung als die bisherigen Befestigungen, wobei die Verbindung auf die genannte Weise nötigenfalls auch einfach und schnell gelöst werden kann.

Demzufolge ist ein späteres Auswechseln des abgenutzten Einsatzes wesentlich einfacher durchführbar und bedeutet einen eine wesentlich geringe operative Belastung darstellenden Eingriff als ein "herkömmlicher". Das neue Verfahren vermindert zufolge der Verkürzung der Operationszeit auch die Zahl der infektionsbedingten Komplikationen.

Trotz alldem schliesst die erfindungsgemässe Konstruktion eine gemeinsame Anwendung der bereits zuvor beschriebenen sonstigen Ausführungslösungen keineswegs aus. Nötigenfalls kann der Mantel des Pfannenkorbes mit einem porösen bzw. bioaktiven Material überzogen und im Verlaufe der Implantation auch ein Klebstoff verwendet werden, obwohl nur dies in extremen Fällen zu empfehlen ist, da Ziel der Konstruktion eben die sichere mechanische Fixierung ist.

Obwohl als Beispiel lediglich eine Hüftgelenksprothese und nur einige Ausführungsformen dieser selbst vorgeführt wurden ist es für den Fachmann offensichtlich, dass die erfindungsgemässe Konstruktion auch bei sonstigen Prothesen günstig eingesetzt werden kann, da sie über sämtliche vorstehend beschriebene günstige Eigenschaften verfügt.

Offensichtlich können die zur Fixierung dienenden Anker auch in sonstigen von den vorgeführten abweichenden anderen Varianten in Abhängigkeit von den gegebenen Forderungen hergestellt werden. Zweckdienlicherweise können die Anker in zahlreichen Varianten und Grössen gefertigt und so in jedem einzelnen Falle deren zweckdienlichsten Varianten eingesetzt werden, da die Anker austauschbar sind. Gegebenenfalls können — wie bereits vorhergehend erwähnt — in einer Prothesenart auch mehrere verschiedene Ankerarten eingesetzt werden, soweit dies die Umstände bedingen.

Offensichtlich ist auch, dass sowohl die Gelenkpfanne als auch der Pfannenkorb in mehreren unterschiedlichen Ausführungsformen gefertigt werden können und das gleiche gilt auch für die zur Fixierung dienende Vorrichtung. Zu betonen ist, dass die vorgeführte Vorrichtung nicht die einzig mögliche Lösung darstellt und dass zur Fixierung der Konstruktion nicht einmal unbedingt ein Werkzeug erforderlich ist, da gegebenenfalls die Gelenkpfanne selbst so ausgeführt werden kann, /z.B. aus Kunststoff hoher Härte bzw., aus keramischem Material/ dass sie das Herausschieben der Anker selbst vornimmt. Zur Erleichterung der Befestigung können auch mit äusseren Metallglocken ausgerüstete Gelenkpfannen gefertigt werden.

Das wesentliche sämtliche aufgezählter bzw. vorstehend nicht erwähnter Varianten jedoch ist, die in den beigelegten Patentansprüchen beschriebene mit Ankern ausgerüstete Konstruktion, die die sichere mechanische Befestigung ermöglicht.

**Patentansprüche**

1. Klebstofflose Gelenkpfannenkonstruktion für Prothesen, insbesondere Hüftgelenksprothesen mit aus gewebefreundlicher Metalllegierung gefertigtem kegelförmigem Pfannenkorb und zur Befestigung der Gelenkpfanne geeignetem Bauelement bzw. Bauelementen, dadurch gekennzeichnet, dass der Pfannenkorb (1) mit in der Mantellinie verlaufenden Aussparungen (3) versehen ist und in den Aussparungen (3) L-förmige Anker (4) um die zur Tangente der Mantellinie des Pfannenkorbes (1) parallel geometrische Achslinie schwenkbar /kippbar/ so eingefügt sind, dass der kürzere Teil der Anker (4) bei dem den kleineren Durchmesser aufweisenden Ende des Pfannenkorbes (1) angeordnet ist und an seinem äusseren Ende als spitze bzw. scharfe Kralle (7) ausgebildet ist, wobei die Stärke seines längeren Teiles bzw. Schaftes (6) gleich der Wandstärke des Pfannenkorbes oder kleiner als diese seine Länge hingegen gleich der Länge der Aussparung (3) ist.

2. Gelenkpfannenkonstruktion nach Anspruch 1, dadurch gekennzeichnet, dass die Seitenwände der in dem Pfannenkorb (1) befindlichen Aussparungen parallel zur Radiusrichtung verlaufen.

3. Gelenkpfannenkonstruktion nach Anspruch 1 oder 2, dadurch gekennzeichnet, dass die zum Boden zuliegende Fläche der im Pfannenkorb (1) befindlichen Aussparungen (3) bogenförmig ausgebildet ist.

4. Gelenkpfannenkonstruktion nach einem der Ansprüche 1 bis 3, dadurch gekennzeichnet, dass an der zum grösseren Durchmesser des Pfannenkorbes (1) zuliegenden Fläche der Aussparungen (3) eine Bettungsrippe (10) vorgesehen ist.

5. Gelenkpfannenkonstruktion nach Anspruch 4, dadurch gekennzeichnet, dass am unteren Ende des Schaftes (6) der Anker (4) eine Bettungsrippe bzw. Bettungsrinne vorgesehen ist.

6. Gelenkpfannenkonstruktion nach einem der Ansprüche 1 bis 5, dadurch gekennzeichnet, dass auf der zur Öffnung des Pfannenkorbes (1) zuliegenden Fläche der Aussparungen (3) ein konvexes eine zur Richtung der Tangenten der äusseren Mantelflächen parallele geometrische Achslinie aufweisende Zylindermantelsegment vorgesehen ist.

7. Gelenkpfannenkonstruktion nach einem der Ansprüche 1 bis 6, dadurch gekennzeichnet, dass an der unteren Fläche des Schaftes (6) der Anker ein konkaves, eine zur Längsachslinie der Kralle (7) senkrechte geometrische Achslinie aufweisendes Zylindermantelsegment vorgesehen ist.

8. Gelenkpfannenkonstruktion nach einem der Ansprüche 1 bis 7, dadurch gekennzeichnet, dass an den Ankern (4) mehrere Krallen (7) ausgebildet sind.

9. Gelenkpfannenkonstrution nach einem der Ansprüche 1 bis 8, dadurch gekennzeichnet, dass die an den Ankern (4) befindliche/n/ Kralle/n/ (7) mehrkantig sind und/oder mehrere Spitzen haben.

10. Gelenkpfannenkonstruktion nach einem der Ansprüche 1 bis 9, dadurch gekennzeichnet, dass die Fläche der Kralle bzw. Krallen (7) mit Nuten (17) und/oder Rippen versehen sind.

11. Gelenkpfannenkonstruktion nach einem der Ansprüche 1 bis 10, dadurch gekennzeichnet, dass die untere Fläche (9) der Krallen (10) bogenförmig ausgebildet ist.

12. Gelenkpfannenkonstruktion nach einem der Ansprüche 1 bis 10, dadurch gekennzeichnet, dass die untere Fläche (19) der Krallen (7) spiralförmig ausgebildet ist.

13. Gelenkpfannenkonstruktion nach einem der Ansprüche 1 bis 12, dadurch gekennzeichnet, dass der Pfannenkorb (1) innen kegelförmig ausgebildet ist und in seinem Boden eine zentral angeordnete Gewindebohrung (2) vorgesehen ist.

14. Gelenkpfannenkonstruktion nach einem der Ansprüche 1 bis 13, dadurch gekennzeichnet, dass die Gelenkpfanne (1) mit einem sich in die Gewindebohrung (2) im Boden des Pfannenkorbes (1) hineinfügenden Gewindezapfen (13) versehen ist.

15. Vorrichtung zur Befestigung der Gelenkpfannenkonstruktion nach einem der Ansprüche 1 bis 14, in der Gelenkgrube, dadurch gekennzeichnet, dass sie aus einer an ihrem einen Ende mit einem sich in die Gewindebohrung (2) im Boden des Pfannenkorbes (1) einfügenden Gewindekopfteil (20) an ihrem anderen Ende mit einem Griff (21) versehenen Welle (19) besteht, auf diese Welle (19) eine auf dem Schraubgewinde (22) sitzenden Rohrwelle (23) aufgezogen ist, wobei an dem zum Griff (21) zuliegenden Ende der Rohrwelle (23) ein Antriebsrad (25), an dem sich an den Pfannenkorb (1) anschliessenden Ende hingegen ein bogenförmiger bzw. eine Kugelfläche aufweisender Spanneinsatz (24) angeordnet ist.

**Revendications**

1. Structure sans adhésif de cuvette ou cupule d'articulation pour prothèses, en particulier pour prothèses d'articulation de hanche, comportant un panier de cuvette conique réalisé en un alliage métallique compatible avec un garnissage et un ou plusieurs élément(s) modulaire(s) apte(s) à la fixation de la cuvette d'articulation, caractérisée en ce que le panier de cuvette (1) est muni d'évidements (3) orientés selon les génératrices d'enveloppe et, dans ces évidements (3), de grapins (4) en forme de L qui sont insérés oscillants ou pivotants autour de la ligne axiale géométriquement parallèle à la tangente à la ligne d'enveloppe du panier de cuvette (1), de telle façon que la partie la plus courte du grapin (4) soit disposée à l'extrémité du panier de cuvette (1) présentant le plus petit diamètre et soit conformée, à son extrémité extérieure, en forme de griffe (7) pointue ou affûtée, l'épaisseur de sa partie la plus longue ou de sa tige (6) étant identique à l'épaisseur de paroi du panier de cuvette ou inférieure à celle-ci, tandis que sa longueur est par contre identique à celle de l'évidement (3).

2. Structure de cuvette d'articulation selon la revendication 1, caractérisée en ce que les parois latérales des évidements se trouvant dans le panier de cuvette (1) sont parallèles à la direction radiale.

3. Structure de cuvette d'articulation selon la revendication 1 ou 2, caractérisée en ce que la surface orientée vers le fond des évidements (3) se trouvant dans le panier de cuvette (1) est cintrée.

4. Structure de cuvette d'articulation selon l'une des revendications 1 à 3, caractérisée en ce qu'elle comporte une nervure d'assise (10) sur la surface des évidements (3) située du côte de grand diamètre du panier de cuvettee (1).

5. Structure de cuvette d'articulation selon la revendication 4, caractérisée en ce qu'elle comporte, à l'extrémité inférieure de la tige (6) du grapin (4), une nervure d'assise ou une rainure d'assise.

6. Structure de cuvette d'articulation selon l'une des revendications 1 à 5, caractérisée en ce qu'elle comporte sur la surface des évidements (3) dirigée vers l'ouverture du panier de cuvette (1), un segment convexe d'enveloppe de cylindre dont la ligne d'axe géométrique est parallèle à la direction de la tangente à la surface d'enveloppe extérieure.

7. Structure de cuvette d'articulation selon l'une des revendications 1 à 6, caractérisée en ce qu'elle comporte un segment concave d'enveloppe cylindrique sur la surface inférieure de la tige (6) du grapin, ce segment présentant une ligne d'axe géométrique perpendiculaire à la ligne d'axe longitudinale de la griffe (7).

8. Structure de cuvette d'articulation selon l'une des revendications 1 à 7, caractérisée en ce qu'elle comporte plusieurs griffes (7) sur le grapin (4).

9. Structure de cuvette d'articulation selon l'une des revendications 1 à 8, caractérisée en ce que la ou les griffe(s) (7) du grapin (4) sont polygonales et/ou présentent plusieurs pointes.

10. Structure de cuvette d'articulation selon l'une des revendications 1 à 9, caractérisée en ce que la surface de la ou les griffe(s) (7) est munie de rainures (17) et/ou de nervures.

11. Structure de cuvette d'articulation selon l'une des revendications 1 à 10, caractérisée en ce que la surface inférieure (9) des griffes (10) présente une forme arquée.

12. Structure de cuvette d'articulation selon l'une des revendications 1 à 10, caractérisée en ce que la surface inférieure (19) des griffes (7) présente une forme en spirale.

13. Structure de cuvette d'articulation selon l'une des revendications 1 à 12, caractérisée en ce que le panier de cuvette (1) présente une forme intérieure conique et est muni, au fond, d'un alésage central fileté (2).

14. Structure de cuvette d'articulation selon l'une des revendications 1 à 13, caractérisée en ce que la cuvette d'articulation (1) est munie d'un tampon fileté (13) inséré dans l'alésage fileté (2) au fond du panier de cuvette (1).

15. Dispositif pour la fixation de la structure de cuvette d'articulation selon l'une des revendications 1 à 14, dans la cavité d'articulation, caractérisé en ce qu'il se compose, à l'une de ses extrémités, d'une pièce de tête filetée (20) insérée dans l'alésage fileté (2) au fond du panier de cuvette (1), et à son autre extrémité, d'un arbre (19) muni d'une poignée (21), un arbre tubulaire (23) étant monté sur le filetage (22) de cet arbre (19), et en ce qu'à l'extrémité de l'arbre tubulaire (23) adjacente à la poignée (21) est disposée une roue ou volant d'entraînement (25), tandis qu'à l'extrémité raccordée au panier de cuvette (1), est disposée une pièce de fixation (24) présentant une surface en voûte ou sphérique.

**Claims**

1. Adhesive-less articular socket construction for prostheses, especially for hip prostheses, having a conical socket basket made of a tissue-compatible metal alloy, and an element respectively elements for fixing the socket, characterised in that the socket basket (1) is provided with cutouts (3) running in the direction of the generatrix and L-shaped anchors (4) are inserted into the cutouts (3) so as to be tiltable around the geometric axis running parallel with the tangent of the generatrix of the socket basket (1) and in such a manner that the shorter parts of the anchors (4) are arranged at the end with the smaller diameter of the socket basket and on the outer end they are formed as a pointed sharp claw (7), wherein the thickness of the longer part respectively shank (6) corresponds to the wall-thickness of the socket basket (1) or it is less than the same, while the length of said longer parts corresponds to the length of the cutout (3).

2. The articular socket construction according to claim 1, characterised in that the lateral walls of the cutouts (3) on the socket basket (1) are parallel with the radial direction.

3. The articular socket construction according to claim 1 or 2, characterised in that the plane of the cutout (3) on the socket basket (1) facing the bottom is arcuate.

4. The articular socket construction according to one of the claims 1 to 3, characterised in that on the plane of the cutouts (3) facing the larger diameter of the socket basket (1) there is an embedding rib (10).

5. The articular socket construction as claimed in claim 4, characterised in that on the lower end of the shanks (6) of the anchors (4) there is an embedding rib or an embedding groove.

6. The articular socket construction according to one of the claims 1 to 5, characterised in that on the plane of the cutouts (3) facing the opening of the socket basket (1) there is a convex cylinder mantle segment and the geometric axis thereof is running parallel with the direction of the tangent of the outer mantle surface.

7. The articular socket construction according to one of the claims 1 to 6, characterised in that on the bottom plane of the shank (6) of the anchors (4) there is a concave cylinder mantle segment, the geometric axis of which is vertical to the longitudinal axis of the claw (7).

8. The articular socket construction according to one of the claims 1 to 7, characterised in that the anchors (4) are formed with a plurality of claws (7).

9. The articular socket construction according to one of the claims 1 to 8, characterised in that the claw(s) (7) on the anchors (4) are many-edged and/or have several points.

10. The articular socket construction according to one of the claims 1 to 9, characterised in that the surface of the claw(s) (7) are provided with grooves (17) and/or ribs.

11. The articular socket construction according to one of the claims 1 to 10, characterised in that the bottom plane (9) of the claws (7) is arcuate.

12. The articular socket construction according to one of the claims 1 to 10, characterised in that the bottom plane (9) of the claws (7) is helical.

13. The articular socket construction according to one of the claims 1 to 12, characterised in that the socket basket (1) has a conical inside and on the bottom there is a threaded bore (2) centrally arranged.

14. The articular socket construction according to one of the claims 1 to 13, characterised in that the articular socket construction (11) is provided with a threaded pin (13) fitting into the threaded bore (2) on the bottom of the socket basket (1).

15. Device for fixing the articular socket construction according to one of the claims 1 to 14 in the articular cavity, characterised in that on one end it is provided with a threaded head-part (20) fitting into the threaded bore (2) on the bottom of the socket basket (1), while on the other end it consists of a shaft (19) provided with a grip (21), and a tubular shaft (23) — fitting with a screwthread (22) — is pulled on the shaft (19) wherein on the end of the tubular shaft (23) facing the grip (21) there is a driving wheel (25) arranged, while on the end fitting to the socket basket (1) there is a tensioning insert (24) with an arcuate respectively spherical surface.

Fig.1

Fig.2

Fig.3

Fig.4

Fig.5

Fig.6

Fig.10

Fig.7

Fig. 8

Fig.9

Fig.11

Fig.12

EP 0 249 594 B1

Fig.13

Fig.14

4